(19) Europäisches Patentamt
European Patent Office
Office européen des brevets

(11) Veröffentlichungsnummer: **0 179 390**
**A2**

# (12) EUROPÄISCHE PATENTANMELDUNG

(21) Anmeldenummer: 85113124.3

(22) Anmeldetag: 16.10.85

(51) Int. Cl.⁴: C 07 C 101/24
C 07 D 233/64, C 07 C 59/347
A 61 K 31/195, A 61 K 31/405
A 61 K 31/205

(30) Priorität: 19.10.84 DE 3438455

(43) Veröffentlichungstag der Anmeldung:
30.04.86 Patentblatt 86/18

(84) Benannte Vertragsstaaten:
AT CH DE FR GB LI NL SE

(71) Anmelder: Institut Dr. Ziegler
Wyhlenweg 18
CH-4126 Bettingen(CH)

(72) Erfinder: Ziegler, Walter Johann, Dr. phil. nat.
Wyhlenweg 18
CH-4126 Bettingen/Basel(CH)

(74) Vertreter: Patentanwälte Dr. Solf & Zapf
Asamstrasse 8
D-8000 München 90(DE)

## (54) L-Lysin- und L-Histidinpyruvat.

(57) Die Erfindung betrifft neue Pyruvatverbindungen, nämlich L-Lysin- und L-Histidinpyruvat, das Verfahren zur Herstellung dieser Verbindungen durch Umsetzung von Brenztraubensäure mit L-Lysin- bzw. L-Histidin und Präparate, enthaltend L-Lysinpyruvat und/oder L-Histidinpyruvat.

EP 0 179 390 A2

Croydon Printing Company Ltd.

# L-Lysin- und L-Histidinpyruvat

## Beschreibung

Die Erfindung betrifft neue Verbindungen der Brenztraubensäure, nämlich L-Lysinpyruvat und L-Histidinpyruvat. Die Herstellung dieser Verbindungen und die Verwendung dieser Verbindungen zur Prophylaxe bzw. zur Behandlung von Schäden an organischen Geweben des menschlichen und tierischen Körpers, die verursacht werden durch gewebeschädigende Wirkstoffe, die in Arzneimitteln, Nahrungsmitteln oder Genußmitteln enthalten sind. Die betroffenen Gewebe umfassen alle Schleimhautgewebe des Nahrungs- und Verdauungstraktes, des Atmungsapparates von der Nase bis in die Lunge, aber auch Lippen, vaginale und andere Gewebe sowie die Hautepithelien der Körperoberfläche.

Viele Stoffe, insbesondere Arzneimittel, Nahrungsmittel oder Genußmittel, die auf Menschen oder Tiere zur Einwirkung gebracht werden, weisen neben der erwünschten Wirkung auch eine unerwünschte Nebenwirkung auf. Solche Nebenwirkungen treten insbesondere bei entzündungshemmenden Substanzen wie Phenylbutazon, Oxyphenylbutazon und bei Mitteln zur Behandlung von Rheuma auf. Man unterscheidet bei den von Menschen oder Tieren eingenommenen Stoffen bzw. Gemischen nach einer dosisabhängigen Schadwirkung und einer unerwünschten Nebenwirkung, die nicht durch eine Überdosierung hervorgerufen wird. So rufen entzündungshemmende Stoffe, deren Hauptvertreter zum Beispiel die Acetylsalicylsäure ist, als unerwünschte Nebenwirkung Schleimhautschädigungen im Gastro-Intestinalbereich auf. Auch bestimmte Phenylessigsäure-Derivate, zum Beispiel das Dichlofenac-Natrium, das als Wirksubstanz in Rheumamitteln enthalten ist, weist eine zellschädigende Nebenwirkung auf. Da in den meisten Fällen die Präparate über längere

Zeiten und oft mehrmals täglich einzunehmen sind, entsteht durch die unerwünschten Nebenwirkungen der Präparate eine erhebliche Gewebeschädigung, insbesondere eine Schleimhautzerstörung und es kommt zu Bildung von Ulcera.

Aus der DE-OS 31 23 259 ist bekannt, daß Natriumpyruvat als schleimhautschützendes Mittel einsetzbar ist. Bei der Verabreichung von Natriumpyruvat besteht der Nachteil, daß das Schutzmittel in beträchtlichen Mengen Natriumionen enthält, die insbesondere im Falle von Hypertonie unerwünscht sind und durch die Basizität des Natriumpyruvats eine gewisse Reizung der Magenschleimhaut eintritt, die der Schutzwirkung entgegenwirkt. Ein weiterer Nachteil liegt darin, daß der Organismus mit Natriummetallionen durch die Verabreichung von Natriumpyruvat belastet wird.

Der Erfindung liegt daher die Aufgabe zugrunde, eine Substanz zur Verfügung zu stellen, die die Nachteile des Natriumpyruvats nicht aufweist und die insbesondere wirksamer zur Prophylaxe und Therapie von Gewebeschäden, und zwar ganz allgemeiner Art, einsetzbar ist.

Überraschenderweise wurde gefunden, daß eine Verminderung der Zellprotrusionen, die durch zellschädigende Wirkstoffe verursacht werden, erreicht werden kann durch L-Lysinpyruvat, L-Histidinpyruvat oder den Gemischen davon. Diese Verbindungen sind neu. Die Herstellung dieser Verbindungen erfolgt durch Umsetzung von Brenztraubensäure, insbesondere frisch destillierter Brenztraubensäure mit einer wäßrigen Lösung der entsprechenden L-Aminosäure. Das dabei entstehende Reaktionsprodukt wird abgekühlt und anschließend lyophilisiert. Die Summenformel von L-Lysinpyruvat lautet $C_9H_{18}N_2O_5$ und die des L-Histidinpyruvats $C_9H_{13}N_3O_5$.

Die Salze der Brenztraubensäure mit den Aminosäuren Lysin und Histidin zeichnen sich dadurch aus, daß sie in wäßriger Lösung praktisch neutral reagieren. Diese Eigenschaft ist an sich im Sinne einer Pufferwirkung jedoch nicht maßgebend für die gesteigerte Wirkung zur Verhinderung einer Plasmapolypenbildung an den Schleimhäuten. Es ist jedoch von Vorteil, daß die relativ neutralen wäßrigen Lösungen von L-Lysinpyruvat bzw. L-Histidinpyruvat keine Reizung der Magenschleimhaut verursachen.

Es ist überraschend, daß die erfindungsgemäßen Substanzen eine so hervorragende Wirkung gegenüber Zellprotrusionen aufweisen, da Lysin und Histidin keine Glieder des Citratzyklus im Stoffwechsel sind und Lysin und Histidin somit nicht in den Citratzyklus eingespeist werden wie andere Aminosäuren, zum Beispiel Alanin.

Es wurde gefunden, daß durch die Verwendung von L-Lysinpyruvat und/oder L-Histidinpyruvat die oben beschriebenen zellschädigenden bzw. gewebeschädigenden Nebenwirkungen von vielen Arzneimitteln, Genußmitteln oder Nahrungsmitteln praktisch vollständig beseitigt werden können. Das Mengenverhältnis von gewebeschädigender Wirksubstanz zur Schutzsubstanz L-Lysinpyruvat und/oder L-Histidinpyruvat liegt vorzugsweise bei einem Molverhältnis von 1:1-3. Eine gute Schutzwirkung wird bereits bei einem Molverhältnis von 1:1 erreicht.

Die erfindungsgemäßen Substanzen L-Lysinpyruvat und/oder L-Histidinpyruvat können zusammen mit der gewebeschädigenden Substanz, d.h. dem Arzneimittel oder dem Nahrungsmittel oder dem Genußmittel oder auch in getrennter Form verabreicht werden. Das L-Lysinpyruvat und/oder L-Histidinpyruvat kann in Form von Pulvern, Tabletten, Granulaten, Dragees oder in Form einer Lösung verabreicht werden. Die erfindungsgemäßen Substanzen können auch in Form von Pulvern, Tabletten, Granulaten, Dragees oder in Form von Lösungen zusammen mit den Arzneimitteln, die die zellschädigende Wirkung bzw. schleimhautschädigende Nebenwirkung aufweisen, verabreicht werden. Die verabreichten Präparate können daneben noch übliche Bestandteile wie Füllstoffe, Geschmackstoffe, Farbstoffe, Filmbildner und/oder andere Hilfsstoffe enthalten.

Der Anteil an L-Lysinpyruvat und/oder L-Histidinpyruvat in dem verabreichten Präparat liegt vorzugsweise bei einer wirksamen Menge, die ausreicht, um die durch den gewebeschädigenden Wirkstoff in dem verabreichten Arzneimittel verursachten Gewebeschädigungseffekt zu verhindern bzw. im wesentlichen aufzuheben.

Die Erfindung wird anhand der nachfolgenden Beispiele näher erläutert.

Beispiel 1

Herstellung von L-Lysinpyruvat

Zu 0,88 g (10 mmol) frisch destillierter Brenztraubensäure, vorgelegt in einem 100 ml-Laborkolben, wurde unter Rühren tropfenweise eine Lösung von 1,46 g (10 mmol) L-Lysin in 10 ml Wasser gegeben, wobei mit weiteren 5 ml Wasser nachgespült wurde. Nach Beendigung der Umsetzung wurde das Reaktionsgemisch sofort tiefgekühlt und anschließend lyophilisiert. Das erhaltene Produkt wurde zu feinen Flocken zerstoßen und nach Trocknung im Hochvakuum über $P_2O_5$ und KOH wurden 2,34 g (10 mmol) Lysinpyruvat erhalten. Eine Lösung von 100 mg des Präparats in 1 ml Wasser hat einen pH-Wert von 5,0.

IR-Spektrum (KBr): Banden bei 3410, 3000 (breit), 2090, 1620 (breit), 1500, 1390, 1350, 1230, 1165, 1135, 1020, 955, 930, 815, 740 und 660 $cm^{-1}$.

Summenformel: $C_9H_{18}N_2O_5$, MG: 234,25

Strukturformel: $CH_3CO\text{-}COO^- \; H_3\overset{+}{N}\text{-}(CH_2)_4\text{-}CH\begin{smallmatrix}COO^-\\NH_3^+\end{smallmatrix}$

## Beispiel 2

### Herstellung von L-Histidinpyruvat

Das Verfahren gemäß Beispiel 1 wurde wiederholt, jedoch mit dem Unterschied, daß 1,55 g (10 mmol) L-Histidin anstelle von L-Lysin eingesetzt wurde. Man erhält nach der Lyophilisierung und Trockung 2,43 g (10 mmol) L-Histidinpyruvat in Form feiner Flocken. Eine Lösung von 100 mg des L-Histidinpyruvats in einem Milliliter Wasser wies einen pH-Wert von 4,9 auf.

Summenformel: $C_9H_{13}N_3O_5$, MG: 243,22

Strukturformel: $CH_3-CO-COO^-$

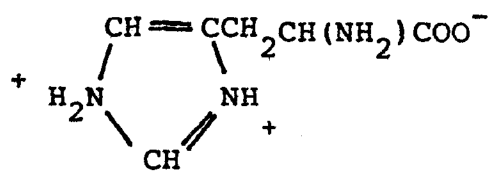

## Beispiel 3

Zur Untersuchung der Wirksamkeit der erfindungsgemäßen Substanzen zur Verhinderung der Zellprotusionsbildung wurden Versuche an Ratten durchgeführt, wobei als schleimhautschädigende Substanz Acetylsalicylsäure verabreicht wurde. Das Ausmaß der Schleimhautschädigung wurde bestimmt durch die Anzahl der gebildeten Plasmapolypen auf der Magenschleimhaut. Die Plasmypolypenbildung, deren Konzentration und Auszählung wurde an Gruppen zu je fünf Sprague Dawley-Ratten von 140-160 g Körpergewicht kontrolliert und die Anzahl der Plasmapolypen auf 1 g Magen (Leergewicht) umgerechnet. Die Tiere waren vor dem Versuch 15-17 h nahrungsfrei. Die Applikation der Testsubstanzen, gelöst in Krebs-Ringer-Lösung (KRL) oder Aqua bidest erfolgte per Schlundsonde. Die Verweildauer der Testsubstanz bis zur Entnahme der Mägen einer Gruppe der

Ratten betrug etwa 20 bis max. 30 min. Dabei wurden dosisabhängig die von der Magenschleimhaut abgeschnürten Plasmapolypen erfaßt. Es wurden fünf Tierversuche - zu einer Serie zusammengefaßt - durchgeführt innerhalb von 40 min, wobei die Mägen wie folgt aufgearbeitet wurden: Jeder Ratte wurden 4,5 ml der Lösung, bestehend aus der in Aqua bidest gelösten Testsubstanz appliziert. Nach einer Einwirkzeit von 15 bis 20 Minuten wurde der Magen entnommen. Der Magen wurde aufgeschnitten, der Inhalt aufgefangen und auf Plasmapolypen geprüft. Der leere Magen wurde in oxygenierte KRL-Lösung mit Glucosezusatz geworfen und dann homogenisiert. Das Homogenisat wurde mit einer Lösung aus einem Copolymeren der Sacharose mit Epichlorhydrin unterschichtet und im Schwingbecherrotor zentrifugiert. Die die Plasmapolypen enthaltende Schicht wurde bei 13000 U/min zentrifugiert, der Überstand wird verworfen und die Plasmapolypen aus dem Sediment ausgezählt.

Es wurden die in der folgenden Tabelle angegebenen Substanzen appliziert und dann die Anzahl der Plasmapolypen auf der Magenschleimhaut aus einer Serie von fünf Tieren als Mittelwert pro Gramm Rattenmagen ermittelt.

| Nr. | Applizierte Substanzen | Anzahl der PP* pro 1 g Rattenmagen |
|---|---|---|
| 1. | 4,5 ml Aq. bidest | ca. 238 000 |
| 2. | 4,5 ml Aq. bidest mit 0,5 mmol L-Lysinpyruvat | ca.230 000 |
| 3. | 4,5 ml Aq. bidest mit 0,5 mmol ASS** | ca. 728 000 |
| 4. | 4,5 ml Aq. bidest mit 0,5 mmol ASS und 0,5 mmol L-Lysinpyruvat | ca. 390 000 |
| 5. | 4,5 ml Aq. bidest mit 0,5 mmol ASS und 0,5 mmol L-Histidinpyruvat | ca. 414 000 |

* PP= Plasmapolypen
**ASS= Acetylsalicylsäure

Die vorstehende Tabelle zeigt, daß durch die gleichzeitige Verabreichung von L-Lysinpyruvat bzw. L-Histidinpyruvat eine ganz erhebliche Verringerung der Plasmapolypenbildung auf bzw. in der Magenschleimhaut erreicht wird, die verursacht wird durch die zellschädigende Acetylsalicylsäure. Bei den durchgeführten Versuchen wurde festgestellt, daß die erfindungsgemäßen Substanzen eine erheblich bessere Schutzwirkung aufweisen als z.B. das bekannte Natrium - pyruvat.

Es wurden analoge Versuche mit der Schadsubstanz 2- (2,6-Dichloranilino)-phenylessigsäure-Natriumsalz durchgeführt. Auch bei diesen Versuchen hat sich gezeigt, daß L-Lysinpyruvat und L-Histidinpyruvat als sehr wirksames Schutzmittel gegen die Bildung von Plasmapolypen einsetzbar sind.

Institut Dr. W. Ziegler

Bettingen/Schweiz

---

L-Lysin- und L-Histidinpyruvat

---

Patentansprüche

1. L-Lysin- und L-Histidinpyruvat

2. Verfahren zur Herstellung von L-Lysinpyruvat, dadurch gekennzeichnet, daß man Brenztraubensäure mit einer wäßrigen Lösung von L-Lysin umsetzt, das Umsetzungsprodukt kühlt und anschließend lyophilisiert.

3. Verfahren zur Herstellung von L-Histidinpyruvat, dadurch gekennzeichnet, daß man Brenztraubensäure mit einer wäßrigen Lösung von L-Histidin umsetzt, das Umsetzungsprodukt kühlt und anschließend lyophilisiert.

4. Präparat zur Prophylaxe und Therapie von Gewebe- bzw. Schleimhautschädigungen, dadurch gekennzeichnet, daß es L-Lysinpyruvat und/oder L-Histidinpyruvat enthält.

5. Präparat nach Anspruch 4, dadurch gekennzeichnet, daß es L-Lysinpyruvat und/oder L-Histidinpyruvat in einer die unerwünschte Nebenwirkung eines verabreichten Arzneimittels, Nahrungsmittels oder Genußmittels im wesentlichen aufhebenden Menge enthält.

6. Präparat nach Anspruch 4 oder 5, dadurch gekennzeichnet, daß es die die Gewebeschädigung hervorrufende Wirksubstanz des Arzneimittels, Nahrungsmittels oder Genußmittels und das L-Lysinpyruvat und /oder L-Histidinpyruvat im Molverhältnis von 1:1-3, insbesondere im Molverhältnis 1:1 enthält.

7. Präparat nach einem der Ansprüche 4 bis 6, dadurch gekennzeichnet, daß es als gewebeschädigende Wirksubstanz Phenylessigsäure-Derivate, insbesondere 2- (2,6- Dichloranilino)-phenylessigsäure, Acetylsalicylsäure oder deren Derivate, Phenylbutazon, Oxyphenylbutazon, Lipidsenker, insbesondere Etofyllinclofibrat und Vincamin und als Schutzsubstanz L-Lysinpyruvat und/oder L-Histidinpyruvat enthält.

8. Präparat nach Anspruch 7, dadurch gekennzeichnet, daß es 2-(2,6-Dichloranilino)-phenylessigsäure und L- Lysinpyruvat bzw. L-Histidinpyruvat im Molverhältnis von 1 : 1,5 bis 2 und ggf. an sich bekannte Streckmittel oder Arzneihilfsstoffe enthält.

9. Verwendung von L-Lysin- und/oder L-Histidinpyruvat als Mittel zur Prophylaxe und Therapie von Gewebeschädigungen, die durch in Arzneimitteln, Genußmitteln oder Nahrungsmitteln enthaltenden Wirksubstanzen verursacht werden.